# EUROPÄISCHE PATENTSCHRIFT

(11) **EP 0 951 301 B1**
(45) Veröffentlichungstag und Bekanntmachung des Hinweises auf die Patenterteilung: **15.10.2003**
(21) Anmeldenummer: 98900064.1
(22) Anmeldetag: 08.01.1998
(51) Int. Cl.: A61M 1/06

(54) **MUTTERMILCHPUMPE**
BREAST MILK PUMP
TIRE-LAIT

(30) Priorität: 10.01.1997 DE 19700545
(43) Veröffentlichungstag der Anmeldung: 27.10.1999
(73) Patentinhaber: NUESCH, LOGISTIK, 9524 ZUZWIL (CH)
(72) Erfinder: NÜESCH, Heinrich, CH-9524 Zuzwil (CH)
(74) Vertreter: Révy von Belvárd, Peter
(86) Internationale Anmeldenummer: CH9800006
(87) Internationale Veröffentlichungsnummer: WO98030257

(56) Entgegenhaltungen:
- EP-A- 0 744 180

## Beschreibung

Die Erfindung bezieht sich auf eine Muttermilchpumpe nach dem Oberbegriff des Anspruches 1. Eine derartige Muttermilchpumpe ist aus der EP-A-0 744 180 bekannt geworden.

Mit der bekannten Muttermilchpumpe wird ein gleichzeitiges, schonendes Absaugen beider Brüste erreicht, wobei der Antrieb elektrisch, z.B. mit einer Batterie als Energiequelle, erfolgen kann. Das Saugen erfolgt dabei zyklisch etwa im natürlichen Rhythmus eines saugenden Babys, und die Zykluszeiten sind an den beiden Brüsten alternierend vorgesehen, so daß die Energiequelle keiner unregelmäßigen Belastung ausgesetzt wird. Maximal soll die Zyklusfrequenz etwa 60 pro Minute betragen, doch hat sich in der Praxis gezeigt, daß eine so hohe Frequenz mit der bekannten Muttermilchpumpe nur schwer erreichbar ist. Dazu kommt, daß der gemeinsame Antrieb, zur Vermeidung einer Synchronisierung der beiden Ventile auch ein ihnen gemeinsames Ventilglied betreibt, das bei höheren Geschwindigkeiten zu Klappergeräuschen neigt. Dies ist nicht zuletzt auch darauf zurückzuführen, daß eine Abstimmung der einzelnen Ventile kaum möglich ist, weshalb die Anordnung auf Fertigungstoleranzen relativ empfindlich ist, d.h. zu große Toleranzen können dazu führen, daß ein Ventil noch nicht schließt, wenn das andere schon offen ist, oder daß es Perioden gibt, in denen beide Ventile schließen, womit dann die gewünschte gleichmäßige Belastung der Energiequelle nicht mehr unter allen Umständen gegeben ist.

Der Erfindung liegt daher die Aufgabe zugrunde, mindestens einen Teil der obigen Nachteile zu vermeiden, und dies geschieht erfindungsgemäß durch die kennzeichnenden Merkmale des Anspruches 1.

Dadurch, daß der Antrieb weiterhin gemeinsam ist, erübrigt sich auch, wie bisher, eine Synchronisierung der beiden Ventile.

Dadurch, daß aber je ein Ventilglied vorgesehen ist, d.h. die Ventilglieder voneinander getrennt sind, reduziert sich ihre Masse, was schon die Klappertendenz vermindert, während es gleichzeitig leichter ist, Herstellungstoleranzen, z.B. durch Auswahl der gesonderten Ventilglieder, auszugleichen.

Eine besonders bevorzugte Ausführungsform wird im Anspruch 3 beschrieben. Eine solche Ausführung vermindert einerseits die Toleranzempfindlichkeit, bringt anderseits eine verbesserte Abdichtung und ermöglicht schließlich auch noch die Ausführungsform nach Anspruch 4.

Weitere Einzelheiten der Erfindung ergeben sich an Hand der nachfolgenden Beschreibung von in der Zeichnung schematisch dargestellten Ausführungsbeispielen. Es zeigen:
- Fig. 1: eine axonometrische Darstellung einer erfindungsgemäßen Muttermilchpumpe, die in
- Fig. 2: in einer Seitenansicht, teilweise im Schnitt zu sehen ist;
- Fig. 2A: einen vergrößerten Ausschnitt aus Fig. 2, der die Ventile im einzelnen zeigt; und
- Fig. 3: eine Sicht im Sinne des Pfeiles III der Fig. 2, jedoch auf eine andere Ausführungsform.

Ein mit einem eine Abschluß- und Lagerwand A' aufweisenden Komponententräger A verbundener Elektromotor 1 treibt, wie beim genannten Stand der Technik, einerseits eine Membranpumpe 2 (oder eine andere Pumpe) an einem Vakuumraum B und anderseits ein Untersetzungsgetriebe 3. Diesbezüglich wird auf die schon genannte EP-A-0 744 180 verwiesen, deren Inhalt hiermit durch Bezugnahme als geoffenbart gelten soll.

Das Untersetzungsgetriebe 3 ist über eine drehfeste Kupplung 5 mit einem Ventilantrieb in Form einer Nockenscheibe 4 verbunden. Diese Nockenscheibe 4 besitzt zwei Nockenkurven 4', die so ausgebildet sind, daß zwei stösselartige Ventilglieder 15 (vgl.

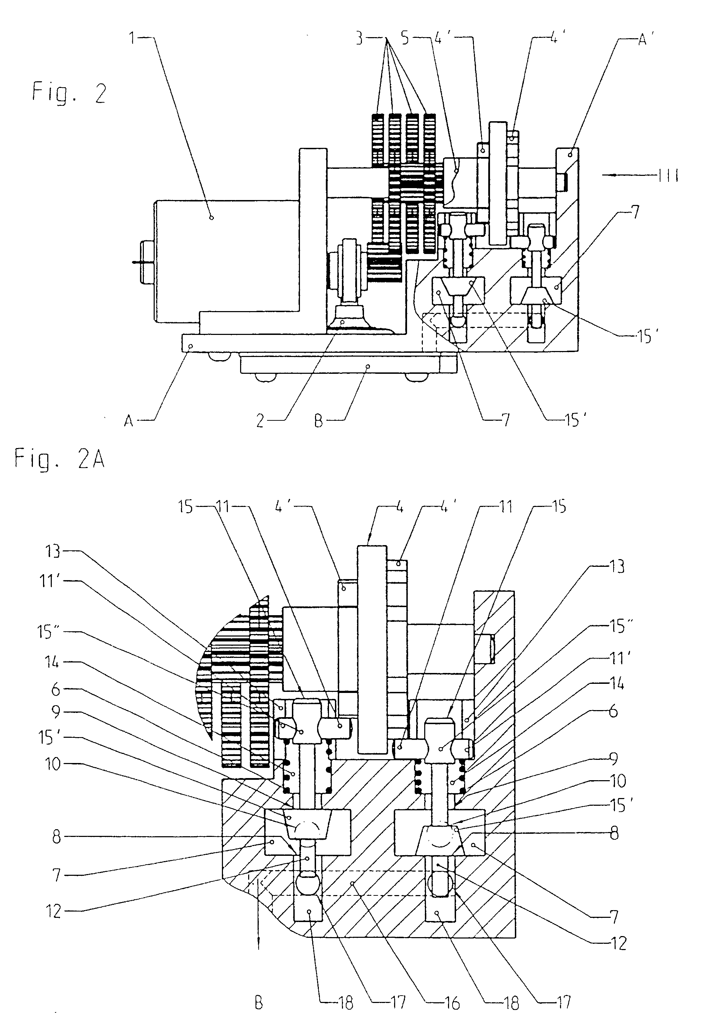

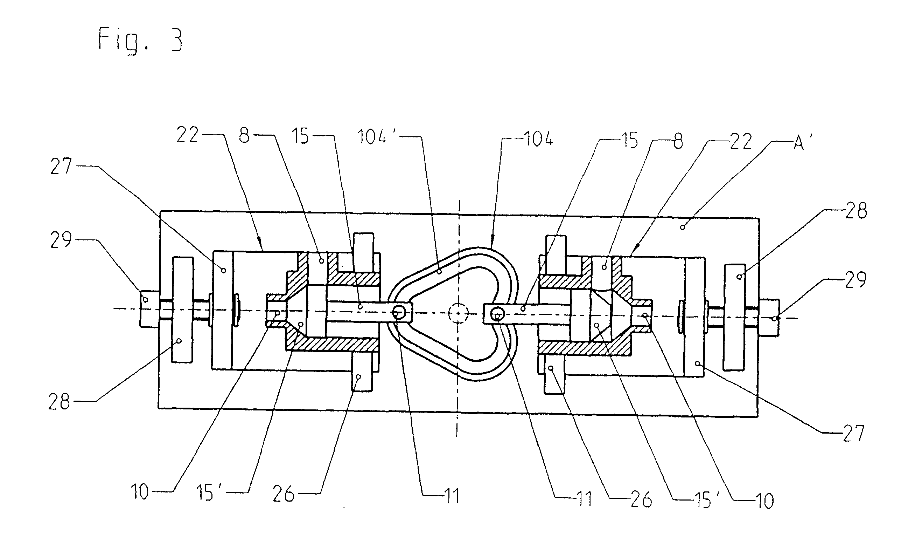

Fig. 2) zueinander gegensinnig bewegt werden. Im vorliegenden Falle liegen die Ventilstössel 15 mit sie durchsetzenden Nokkenfolgerstiften 11 an der Außenfläche der Nocken 4' kraftschlüssig unter dem Drucke von Federn 6 an. Gegebenenfalls kann aber auch eine formschlüssige Führung für die Nockenfolger durch eine Nockennut vorgesehen sein, wie dies später an Hand der Fig. 3 beschrieben wird.

Während das eine Ende des Nockenfolgers 11 an der jeweiligen Nocke 4' anliegt, ist das andere Ende 11' vorzugsweise in einer vertikalen Nut 13 geführt, so daß eine Verdrehung des Ventilstössels 15 ausgeschlossen ist. Gleichzeitig kann sich durch diese etwa kreuzförmige Ausbildung eines Ventilschaftes 12 und des Nockenfolgerstiftes 11 die jeweilige Druckfeder 6 mit einem ihrer Enden abstützen, wogegen das andere Ende am Boden einer erweiterten Bohrung 14 liegt. Der Nockenfolgerstift 11 durchsetzt einen verbreiterten Kopf 15" des Ventilstössels 15. Dieser Kopf 15" kann mit dem Schaft 12 verschraubt sein, so daß die jeweilige Stellung des Ventilgliedes 15 relativ zur Ventilkammer bzw. eines Kolbenteiles 15' relativ zum Antrieb 4 für jedes der beiden dargestellten Ventile justierbar ist.

Jeder Ventilstössel 15 weist ein solches kolbenartig am Schaft 12 befestigtes Dichtelement 15' auf. Dieser Kolbenteil 15' ist bevorzugt aus elastischem Material, wie Silikongummi, gefertigt und somit zusammenpreßbar. Wenn, wie es einer besonders bevorzugten Ausführung entspricht, dieser Kolbenteil 15' entspannt ist, ist seine axiale Erstreckung, d.h. bezogen auf Fig. 2 seine Höhe, mindestens gleich groß, gegebenenfalls sogar etwas größer als die Dimension der Ventilkammer 7 in gleicher Richtung, in welcher er beweglich ist. Dagegen bildet der Kolbenteil 15' in seinen beiden, in Fig. 2 dargestellten Extremlagen jeweils oben bzw. unten einen elastisch zusammengedrückten Wulst 15". Zur Erleichterung dieses Effektes mag es vorteilhaft sein, wenn der Kolbenteil 15' nur in seiner axialen Mitte mit dem Ventilschaft 12 verbunden ist, so daß seine axialen Enden frei sind.

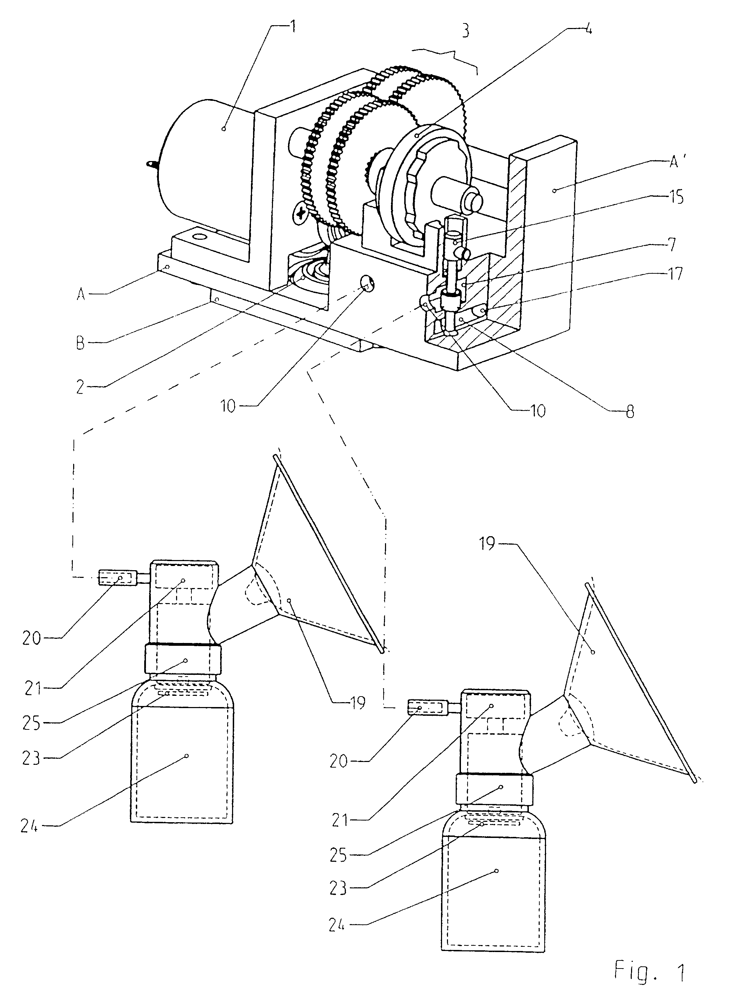

Mit dem Vakuumraum B steht ein Kanal 16 in Verbindung, der im Bereiche jeder Ventilkammer 7 einen Auslaß 17 aufweist. Da der Ventilschaft 12 schmäler ist als die ihn umgebende Bohrung 18, bleibt zwischen ihm und der Innenwand dieser Bohrung 18 ein Ringraum, der mit dem Auslaß 17 jeweils verbunden ist. Die Bohrung 18 führt über einen Eingang 8 in die Ventilkammer 7, aus der eine waagrechte Auslaßbohrung 10 herausführt. Wie Fig. 1 zeigt, können an diese Auslaßbohrungen 10 je ein Schlauch 20 angeschlossen werden, der dann zu einer Saugkammer 21 sowie zum Brustkörper 19 führt. An die Saugkammer ist über ein Rückschlagventil 23 zum Abschluß eines Saugraumes 25 ein Milchsammelbehälter 24 angeschlossen.

Durch die oben genannte besonders bevorzugte Ausbildung mit dem elastischen, großen Kolbenteil 15' werden die nachfolgend beschriebenen drei verschiedenen Schaltstellungen des Ventils ermöglicht.

In einer Offenstellung öffnet der Kolbenteil 15' den Eingang 8 von der Bohrung 18 zur Ventilkammer 7, so daß diese unter den Sog aus dem Vakuumraum B unterhalb der Membranpumpe 2 gerät. Diese Stellung ist in Fig. 2 an Hand des linken Ventils veranschaulicht. Dabei wird die zugehörige Auslaßbohrung 10 vom Kolbenteil 15' freigegeben, so daß der Sog auch an dieser Bohrung 10 sowie am angeschlossenen Schlauch 20 anliegt. Der Kolbenteil 15' verschließt gleichzeitig eine Entlüftungsöffnung 9 an der Oberseite der Ventilkammer 7, also gegenüber der Eingangsöffnung 8. Diese Entlüftungsöffnung 9 dient dazu, mit dem Ende eines Saugzyklus den Saugdruck relativ rasch abzubauen, indem die Auslaßbohrung 10 während des Hubes des Kolbenteiles aus der linken Stellung der Fig. 2 in die rechte Lage mit der Entlüftungsöffnung 9 in Verbindung kommen kann, sofern nicht die später besprochene Ausbildung für eine solche Zwischenstellung vorgesehen ist.

In der in Fig. 2 an Hand des rechten Ventils gezeigten Geschlossenstellung wird der Kolbenteil 15' unter Bildung des schon erwähnten Wulstes 15" fest gegen die Eingangsöffnung 8 gedrückt und sperrt, wie ersichtlich, gegebenenfalls auch die Auslaßbohrung 10 ab. Diese Auslaßbohrung 10 liegt aber, wie Fig. 2 zeigt, teilweise oberhalb des Wulstes 15", so daß gewünschtenfalls eine Verbindung zur Entlüftungsöffnung 9 gegeben sein kann. Das Ausmaß einer solchen Verbindung - und damit der Entlastung des Saugdruckes am jeweiligen Brustkörper 19 (vgl. Fig. 1) kann durch die Anordnung der Auslaßbohrung 10 relativ zur Ventilkammer 7 eingestellt werden. In jedem Falle aber wird in der Geschlossenstellung der Eingang 8 vom Auslaß 10 getrennt; bei Anordnung einer Entlüftungsöffnung 9, wie es bevorzugt ist, aber wird der Auslaß 10 mit dieser verbunden, so daß der auf den Brustkörper 19 (Fig. 1) wirkende Saugdruck praktisch schlagartig abgebaut wird.

In Fig. 2 liegt die Auslaßbohrung 10 auf halber Höhe der Ventilkammer 7, und eine stärkere bzw. raschere Entlüftung kann erreicht werden, indem man die Auslaßbohrung 10 etwas höher oder tiefer anordnet. Es wäre aber auch eine Ausführung denkbar, bei der die Kammerwand mit der Auslaßbohrung 10 als höhenverstellbarer Schieber mit einer Öffnung als Auslaßbohrung ausgebildet ist, die in einen erweiterten Auslaßraum des Komponententrägers A mündet. Durch Verschieben eines solchen Schiebers kann dann die Sogwirkung bzw. die Entlüftung individuell eingestellt werden.

Es wurde oben bereits erwähnt, daß die axiale Erstreckung, d.h. bezogen auf Fig. 2 die Höhe, des Kolbenteiles 15' bevorzugt mindestens gleich groß, gegebenenfalls sogar etwas größer als die axiale Dimension der Ventilkammer 7 sein kann. Ist dies der Fall, so verschließt der Kolbenteil in einer Zwischenstellung zwischen den in Fig. 2 gezeigten Stellungen unter Entspannung elastisch praktisch alle Öffnungen 8, 9 und 10. Da kurz zuvor noch die Eingangsöffnung 8 mit der Auslaßbohrung 10 in Verbindung stand, liegt an letzterer nach wie vor der Saugdruck an, der sich nur langsam, z.B. durch am Brustkörper 19 eintretende Leckluft, abzubauen vermag.

Das bedeutet aber, daß bei einer solchen Ausführung der Saugdruck länger wirksam ist, als es der Fall wäre, wenn der Kolbenteil 15' kleiner bzw. unelastisch wäre. Dies bedeutet aber, daß die vom Motor 1 aufgebrachte Energie besser ausgenützt werden kann und daher das Ventilglied 15 früher in seine Geschlossenstellung (rechts in Fig. 2) zurückkehren kann, da ja der Saugdruck noch für eine Weile erhalten bleibt. Dies aber ermöglicht höhere Zyklusfrequenzen, wie sie beim Stande der Technik zwar angestrebt, in der Praxis aber nicht erreicht worden sind. Das Abpumpen der Milch erfolgt somit weiterhin alternierend unter Schonung der Batterie, es gibt aber Überschneidungsperioden, in denen der Saugdruck am einen Brustkörper 19 noch ansteht, während das Absaugen am anderen Brustkörper bereits beginnt.

Aus den obigen Erläuterungen ist ersichtlich, daß die Anordnung dreier Öffnungen 8, 9 und 10 an der Ventilkammer 7 prinzipiell von Vorteil ist und neuartige Funktionseigenschaften und -möglichkeiten bringt, so daß dieses Konzept, unabhängig von der Verwendung gesonderter Ventilglieder oder eines gemeinsames Ventilgliedes (wie beim Stand der Technik), eine eigene Erfindung darstellt.

Während beim Ausführungsbeispiel nach den Fig. 1 und 2 die Ventilglieder 15 parallel zueinander angeordnet sind, zeigt Fig. 3, daß auch andere Anordnungen möglich sind. Hier liegt eine formschlüssige, gemeinsame Nocke 104 mit einer als Nut 104' ausgebildeten Nockenführung an der Außenseite der Wand A'. In diese Nut 104' greifen wiederum Nockenfolger 11 ein. Die Nocke 104 ist, wie ersichtlich, als Herzkurve ausgebildet, wie sie etwa in der Textilindustrie zur Erzeugung einer hin- und hergehenden Bewegung gebräuchlich ist. Somit werden die einander gegenüberliegend angeordneten Ventilglieder 15 bezüglich ihres jeweils zugehörigen Ventilgehäuses gegenläufige Bewegungen ausführen.

Die Ventilgehäuse sind nach Art von sog. Cartridge-Ventilen konstruiert, die im allgemeinen als AUF-ZU-Ventile, also als Schaltventile, ausgebildet sind. Es sei jedoch erwähnt, daß es auch bekannt ist, solche Ventile als Proportionalventile auszubilden, was im Rahmen dieser Erfindung ebenfalls nicht ausgeschlossen werden soll. Jedenfalls bedeutet die Ausbildung als Cartridge-Ventil, daß beispielsweise ein seitlicher Eingang 8 und ein axialer Ausgang 10 vorgesehen ist, mit denen ein, beispielsweise wiederum elastischer, Kolbenteil 15' zusammenwirkt. Selbstverständlich kann auch hier gewünschtenfalls eine Entlüftungsöffnung am Gehäuse vorgesehen werden.

Um nun eine individuelle Anpassung und Einstellung vornehmen zu können, ist jedes der Gehäuse auf einer Platte 22 gelagert. Durch Befestigen der Gehäuse auf der jeweiligen Platte 22, z.B. durch Kleben, in einem gewählten Abstand zur Getriebewelle 3 kann eine individuelle Einstellung der Öffnungs- und Verschließperioden erreicht werden.

Bevorzugt sind jedoch die Platten 22 an der Wand A' mit Hilfe von aus dieser allmählich hervorragenden Führungen 26 verschiebbar geführt und bilden so eine Art Justierschlitten, der an einem Ende mit einer abgewinkelten Wand 27 versehen ist. An der Wand A' ragen dagegen ortsfeste, z.B. eingegossene, Lappen 28 vor. Von außen zugängliche Justierschrauben 29 sind mit der abgewinkelten Wand 27 axial unverschieblich aber drehbar verbunden und sind gleichzeitig in ein Innengewinde des jeweiligen Lappens 28 eingeschraubt. Somit kann durch Verdrehen der Schrauben 29 der relative Abstand von Ventilgehäuse, Ventilglied 15 und Antrieb 104 in analoger Weise eingestellt werden, wie dies oben für das vorherige Beispiel an Hand der Schraubköpfe 15" erläutert wurde. Dies wird natürlich durch die erfindungsgemäße Trennung der beiden Ventilglieder 15 voneinander bei gleichzeitiger Beibehaltung eines gemeinsamen Antriebes 104 ermöglicht.

Im Rahmen der Erfindung sind zahlreiche Variationen denkbar; so braucht das Dichtelement 15 nicht unbedingt kolbenartig ausgebildet zu sein, da ja - im Falle der Fig. 1 und 2 nur der Bereich der elastischen Wülste 15" dichtend gegen die jeweilige Öffnung 8 bzw. 9 gedrückt wird. Mit gleichem Nutzen könnten bloß diese Wülste 15" durch elastische, zweckmäßig gegen die jeweilige Öffnung zu konkav gewölbte, Teller ersetzt werden.

## Patentansprüche

1. Muttermilchpumpe zum Anschluß zweier etwa trichterförmiger Brustkörper (19) über je eine Saugleitung (10, 20) und je ein Ventil (7, 15) an eine Vakuumquelle (2, B), um Muttermilch mit Hilfe eines gemeinsamen Antriebs (4) in einen Auffangbehälter (24) zu pumpen, **dadurch gekennzeichnet, daß** der die beiden Ventile (7, 15) betätigende gemeinsame Antrieb (4) mit je einem in je einem eine Ventilkammer (7) mit Eingangs- (8) und Auslaßöffnung (10) umfassenden Ventilgehäuse zumindest aus einer offen- in eine Geschlossenstellung bringbaren Ventilglied (15) verbunden ist.

2. Muttermilchpumpe nach Anspruch 1, **dadurch gekennzeichnet, daß** die beiden Ventilgehäuse bzw. Ventilkammern (7) symmetrisch zu beiden Seiten des gemeinsamen Antriebes (4; 104) angeordnet sind.

3. Muttermilchpumpe nach Anspruch 1 oder 2, **dadurch gekennzeichnet, daß** die Ventilglieder (15) jeweils einen an einem Schaft (12) befindlichen verbreiterten, z.B. kolbenartigen, Teil als Dichtungselement (15') aufweisen, und daß dieses Dichtungselement (15') aus elastischem Material, z.B. aus Silikongummi, gefertigt ist.

4. Muttermilchpumpe nach Anspruch 3, **dadurch gekennzeichnet, daß** das Dichtungselement (15') in einem entspannten Zustand eine mindest gleich große oder größere axiale Erstreckung aufweist als die Ventilkammer (7), und das Ventilglied (15) so zwischen der Offenstellung und der Geschlossenstellung eine Zwischenlage einzunehmen vermag, in der alle Öffnungen (8-10) verschlossen sind.

5. Muttermilchpumpe nach Anspruch 3 oder 4, **dadurch gekennzeichnet, daß** die Ventilkammer (7) mit einer nach außen führenden Entlüftungsöffnung (9) zur Absenkung des Saugdruckes verbunden ist, und daß diese Entlüftungsöffnung (9) durch das Ventilglied (15) mindestens in dessen die Eingangs- (8) mit der Auslaßöffnung (10) verbindenden Offenstellung verschließbar ist.

6. Muttermilchpumpe nach Anspruch 5, **dadurch gekennzeichnet, daß** die Entlüftungsöffnung (9) der Eingangsöffnung (8) gegenüberliegt.

7. Muttermilchpumpe nach einem der vorhergehenden Ansprüche, **dadurch gekennzeichnet, daß** eine Justiereinrichtung (12, 15"; 26-29) zum Einstellen der relativen Lage von Ventilgehäuse, Ventilglied (15) und Antrieb (4; 104) vorgesehen ist.

## Claims

1. Breast milk pump for attaching two approximately funnel-shaped breast bodies (19) each via a suction line (10, 20) and a valve (7, 15) to a vacuum source (2, B) in order to pump breast milk with the aid of a common drive (4) into a collecting container (24), **characterised in that** the common drive (4) actuating the two valves (7, 15) is connected, in each case, to a valve member (15) switchable at least from an open position into a closed position, each in a valve housing including a valve chamber (7) with an inlet opening (8) and outlet opening (10).

2. Breast milk pump according to Claim 1, **characterised in that** the two valve housings and valve chambers (7) are arranged symmetrically on either side of the common drive (4; 104).

3. Breast milk pump according to Claim 1 or 2, **characterised in that** the valve members (15) each have a broadened, for instance, piston-like member situated on a shaft (12), as a sealing element (15'), and that this sealing element (15') is made from elastic material, for instance, silicone rubber.

4. Breast milk pump according to Claim 3, **characterised in that** in a relaxed condition, the sealing element (15') has an axial extent that is at least as large as or larger than that of the valve chamber (7), and the valve member (15) is therefore able to assume an intermediate position, between the open position and the closed position, where all the openings (8-10) are closed.

5. Breast milk pump according to Claim 3 or 4, **characterised in that** the valve chamber (7) is connected to a ventilation opening (9) leading to the outside for reducing the suction pressure and that this ventilation opening (9) may be closed by the valve member (15) at least in its open position connecting the inlet opening (8) with the outlet opening (10).

6. Breast milk pump according to Claim 5, **characterised in that** the ventilation opening (9) lies opposed to the inlet opening (8).

7. Breast milk pump according to one of the previous claims, **characterised in that** an adjustment device (12, 15 " ; 26-29) for adjusting the relative position of the valve housing, the valve member (15) and the drive (4; 104) is provided.

## Revendications

1. Tire-lait pour la connexion de deux corps de sein (19) approximativement en entonnoirs, chacun par une conduite d'aspiration (10, 20) et un soupape (7, 15) à une source à vide (2, B), pour pomper du lait maternel dans un collecteur (24) au moyens d'un entraînement commun (4), **caractérisé en ce que** l'entraînement commun (4) actionnant les deux soupapes (7, 15) est relié à un élément de soupape (15) respective, chacun capable à être amené d'une position d'ouverture à une position de fermeture et chacun situé dans un boîtier de soupape, qui comprend une chambre de soupape (7) ayant une ouverture d'entrée (8) et une ouverture de décharge (10).

2. Tire-lait selon la revendication 1, **caractérisé en ce que** les deux boîtiers de soupape ou chambres de soupape (7) sont disposé symétriquement des deux côtés de l'entraînement commun (4; 104).

3. Tire-lait selon la revendication 1 ou 2, **caractérisé en ce que** les éléments de soupape (15) comprennent comme élément de bourrage (15') chacun une partie élargie, par exemple en forme de piston, située à une tige (12), et que cet élément de bourrage (15') est produit d'un matériau élastique, par exemple de caoutchouc silicone.

4. Tire-lait selon la revendication 3, **caractérisé en ce que** l'élément de bourrage (15'), dans une condition détendue, a une dimension axiale, qui est au moins égale ou plus grande que la chambre de soupape (7), et qu'ainsi l'organe de soupape (15) est capable d'occuper une position intermédiaire entre la position d'ouverture et la position de fermeture, dans laquelle tous les ouvertures (8-10) sont fermées.

5. Tire-lait selon la revendication 3 ou 4, **caractérisé en ce que** la chambre de soupape (7) est reliée à une ouverture d'aération (9) conduisant à l'extérieur pour la réduction de la pression d'aspiration, et que cette ouverture d'aération (9) peut être fermée par l'organe de soupape (15) au moins dans sa position d'ouverture, dans laquelle l'ouverture d'entrée (8) est an communication avec l'ouverture de décharge (10).

6. Tire-lait selon la revendication 5, **caractérisé en ce que** l'ouverture d'aération (9) est opposée à l'ouverture d'entrée (8).

7. Tire-lait selon une quelconque des revendications précédents, **caractérisé en ce qu'**un dispositif d'ajustage (12, 15"; 26-29) est prévu pour ajuster la position relative du boîtier de soupape, de l'organe de soupape (15) et de l'entraînement (4; 104).
